# EUROPEAN PATENT SPECIFICATION

(11) **EP 0 879 035 B1**
(45) Date of publication and mention of the grant of the patent: **14.06.2006**
(21) Application number: 96911535.1
(22) Date of filing: 01.04.1996
(51) Int. Cl.: A61K 9/00, A61K 33/24, A61K 31/29, A61Q 11/00

(54) **BISMUTH-CONTAINING COMPOSITIONS IN TOPICAL DOSAGE FORMS**
BISMUT ENTHALTENDE ARZNEIMITTEL IN TOPISCHEN DARREICHUNGSFORMEN
COMPOSES CONTENANT DU BISMUTH POUR ADMINISTRATION TOPIQUE

(30) Priority: 24.08.1995 US 518971; 08.12.1995 WO PCT/US95/15985; 31.01.1996 US 594871; 31.01.1996 US 594148
(43) Date of publication of application: 25.11.1998
(60) Divisional application: 06007503.3
(73) Proprietor: Josman Laboratories, Inc., Orange, CA 92667 (US)
(72) Inventor: Athanikar, Narayan Krishnarao, Orange, CA 92667 (US)
(74) Representative: Setna, Rohan P.
(86) International application number: PCT/US1996/004495
(87) International publication number: WO 1997/007757

(56) References cited:
- EP-A- 0 075 992
- WO-A-97/00668
- DE-A- 3 127 639
- GB-A- 2 195 248
- US-A- 3 824 006
- US-A- 3 929 449
- US-A- 4 514 421
- US-A- 4 652 444
- US-A- 4 879 116
- US-A- 5 017 367
- US-A- 5 196 205
- US-A- 5 264 222
- US-A- 5 294 433
- US-A- 5 372 815
- DATABASE WPI Section Ch, Week 199613 Derwent Publications Ltd., London, GB; Class B05, AN 1996-124072 XP002176392 & JP 08 020543 A (KAIGAI SEIYAKU KK), 23 January 1996 (1996-01-23)
- TIOMNY E ET AL: "Halitosis and Helicobacter pylori. A possible link?." JOURNAL OF CLINICAL GASTROENTEROLOGY, (1992 OCT) 15 (3) 236-7. , XP001012914
- NGUYEN A M ET AL: "Detection of Helicobacter pylori in dental plaque by reverse transcription-polymerase chain reaction." JOURNAL OF CLINICAL MICROBIOLOGY, (1993 APR) 31 (4) 783-7. , XP000905084
- MORRIS A ET AL.: "Treatment of Campylobacter-pylori gastritis: a pilot study using pirenzepine dihydrochloride (Gastrozepin) and three formulations of colloidal bismuth subcitrate (De-Nol)" NEW ZEALAND MEDICAL JOURNAL, vol. 101, no. 856 part 1, 1998, pages 651-654, XP001012765
- THERAPY REVIEW SCAND. J. GASTROENTEROL., 1989, Vol. 24 (Suppl. 160), AXON, "Campylobacter Pylori", pages 35-38. XP002963163
- JOURNAL OF CLINICAL MICROBIOLOGY, April 1993, Vol. 31, No. 4, NGUYEN et al., "Detection of Helicobacter Pylori in Dental Plaque by Reverse Transcription-Polymerase Chain Reaction", pages 783-787. XP000905084

## Description

### BACKGROUND OF THE INVENTION

Until recently, excessive gastric acidity and mental stress were thought to be major pathophysiological reasons for occurrence of peptic ulcers. In the early 1980, Marshall and Warren (Warren, Lancet, 1:1273-1275, 1983 and Marshall et al, Lancet, 2:1311-1315, 1984) first reported an unidentified curved bacilli in the stomach of patients with gastritis and peptic ulcers. These bacilli, which later were identified as a gram negative spiral bacterium and named Helicobacter pylori (Goodwin et al. ; Int. J. Syst. Bacteriol. 39:397-405, 1989), have been demonstrated to be associated with gastritis and peptic ulcers (Buck et al., J. Infect. Dis. 153:664-669, 1986 and Graham, Gastroenterology 96:615-625, 1989), and are thought to be transmitted by person-to-person contact.

Recent clinical investigations have shown a definitive presence of H. Pylori in the dental plaque (Nguyen et al., Journal of Clinical Microbiology 31(4):783-787, 1993; Desai et al., Scandinavian Journal of Gastroenterology 26:1205-1208, 1991; and Lambert et al., Lancet 341(8850):957, 1993), and have also shown that standard oral hygiene practice does not help reduce H. Pylori presence in the oral cavity (Nguyen et al., Journal of Clinical Microbiology 31(4):783-787, 1993). As a result of these recent discoveries associating bacterial infection in the causation of peptic ulcer disease, questions regarding the previously established paradigms of ulcer treatment and healing processes have been raised.

H₂ receptor blockers which suppress acid secretion, such as cimetidine (Tagamet® ) and ranitidine (Zantac® ), have been used to treat and heal duodenal ulcers (Jones et al., Gut. 28:1120-1127, 1987; McIsaac et al., Aliment. Pharmacol. Therap. 1:369-381, 1987; and Boyed et al., Amsterdam:Excerpta Medica, 14-42, 1984). Recently, however, a number of clinical investigations have demonstrated that 70-80% of healed duodenal ulcers reoccur within the next year (Goodwin et al., Int. J. Syst. Bacteriol 39:397-407, 1989), and that these drugs do not reverse the tendency for ulcers to form (Wormsley, British Medical Journal 293:1501, 1986; Gudman et al., British Medical Journal i:1095-1097, 1978; and Bardhan et al., British Medical Journal 284:621-623, 1982).

For many years, bismuth compounds have been used for treating ulcers. Clinical investigations comparing the efficacy of CBS (also known as tripotassium dicitrato bismuthate (TDB)) with placebo (Lambert, Scandinavian Journal of Gastroenterology 26(Supplement 185):13-21, 1991), cimetidine (Bianchi, et al., Lancet 2:698, 1984), and ranitidine (Bianchi et al., Gut. 25:565, 1984; Lee et al., Lancet 1:1299-1301, 1985; and Dobrilla et al., Gut. 29:181-187, 1988) in initial healing and relapse rates of duodenal ulcers, have shown significantly lower relapse rates in patients treated with CBS. The therapeutic efficacy of CBS (and other bismuth compounds), in healing duodenal ulcers and lowering relapse rates, is attributed to its specific antibacterial activity against H. Pylori (McNutty et al., Antimicrobial Agents Chemotherapy 28:837-838, 1985; Lambert et al., Antimicrob. Agents Chemotherapy 3:510-511, 1986; and Goodwin et al., J. of Antimicrobial Agents Chemotherapy 17:309-314, 1986). The minimum inhibitory concentration (MIC) for CBS against H. Pylori is reported to be 8 mg/L (Lambert et al., Antimicrob. Agents Chemotherapy 3:510-511) and the range is 4-32 mg/L (Lambert et al., Antimicrob. Agents Chemotherapy 3:510-511).

In addition to its bacteriocidal activity, CBS has been demonstrated to enhance mucus glycoprotein secretion, strengthen viscoelastic gel properties of mucus, cause increased concentration of epithelial growth factor (EGF) in ulcer tissue, and stimulate prostaglandin synthesis in the gastric antral mucosa (Lee, Scandinavian Journal of Gastroenterology 26(Supplement 185):1-6, 1991). These gastroprotective properties of CBS may contribute to the initial healing of duodenal ulcers and the observed lower rates of relapse by returning the gastric mucosal cells to normal physiologic function. The gastroprotective effects of CBS in prevention of gastric lesions induced by various ulcerogenic agents and the mechanism of ulcer healing have been demonstrated in animal studies (Konturek et al., Digestion 37(Supplement 2):8-15, 1987 and Konturek et al., Scandinavian Journal of Gastroenterology 21(Supplement 122):6-10, 1986).

Because of the finding that bismuth is an effective antibacterial agent against H. Pylori, concomitant dosages of bismuth-containing compounds with other anti-ulcer drugs have been increasingly applied in many clinical cases for treatment of peptic ulcers. The most commonly used regiments include double or triple therapy with bismuth; meanwhile, some recent reports regarding quadruple therapy (wherein a proton pump inhibitor is added to triple therapy) have shown eradication rates of over 90 % , but also cause severe side effects such as vomiting and diarrhea.

Additionally, while antibacterial therapy (bismuth and amoxycillin or doxycycline) was shown to be effective in eliminating H. Pylori from the gastric mucosa of duodenal ulcer patients, this therapy had no effect on the H. Pylori colonies in their dental plaque (Desai et al., Scandinavian Journal of Gastroenterology 26:1205-1208, 1991, Nguyen et al., Journal of Clinical Microbiology 31(4):783-787, 1993). The continued presence of H. Pylori in the dental plaque raises the question of whether the relapse of duodenal ulcers is inevitable (Desai et al., Scandinavian Journal of Gastroenterology 26:1205-1208, 1991 and Abraham et al., Indian Journal of Gastroenterology 9(4):265-6, Editorial, 1990).

Triple therapy, consisting of an antibiotic (amoxicillin, tetracycline or erythromycin), metronidazole, and bismuth compounds, has been reported to result in more than a 95% eradication rate for H. Pylori, and reduced ulcer relapse rate to less than 10% during a 12-month follow-up period (Graham et al., Gastroenterology 102:493-496, 1992 and Borody et al., Gastroenterology 102:A 44, 1992). It is interesting to note that metronidazole as a single agent has only 5% eradication rate for H. Pylori, but as a component of triple therapy, it increases the eradication rate to as high as 95%. When metronidazole-resistant strains of H. Pylori are encountered (about 25% of the H. Pylori strains are resistant), the eradication rate falls to about 50% (Logan et al., Lancet 338:1249-1252, 1991).

One possible explanation for this observed clinical efficacy of metronidazole in combination therapy is that metronidazole is actively secreted in the saliva (Mustofa et al., International Journal of Clinical Pharmacology, Therapy, and Toxicology 29(12):474-478, 1991) where it might be exerting its antimicrobial action against dental plaque-bound H. Pylori colonies. The typical steady state saliva represent 10 to 20 times the MIC for H. Pylori. Another antibiotic, Clarithromycin, a new-generation macrolide, which has shown a 40 to 60% cure rate as a single agent, is also secreted in the saliva. Therefore, it is reasonable to believe that in order to achieve nearly complete eradication of H. Pylori, and prevent peptic ulcer relapse, eradication of this organism from the oral cavity is essential. Colloidal bismuth subcitrate (CBS), the most effective single agent against H. Pylori, is however not absorbed significantly from the GI, and therefore, produces no salivary concentrations. But as a single agent, it is about 6 to 8 times more effective in eradicating H. Pylori than metronidazole. The present invention therefore is related to development of a therapeutic modality to effectively eradicate H. Pylori reservoir from the oral site.

Tiomny et al. (J. Clin. Gastroenterol. 1992, 15(3): 236-7) studied the use of tablets of colloidal bismuth subcitrate and metronidazole for the treatment of halitosis.

Furthermore, recent clinical studies have implicated this insidious organism in gastric cancer (Parsonnet, Gastroenterology Clinics of America, Helicobacter pylori Infection, Dooley CP, Cohen, H. Guest Editors, Volume 22, No.1, pp. 89-104, March 1993). A progression of gastric pathology from gastritis and ulcers to cancer involving H. Pylori has been described (Recavarren-Arie et al., Scandinavian Journal of Gastroenterology 26 (Supplement 181):51-57, 1991). In addition to H. Pylori infection, low concentration levels of ascorbic acid in the gastric mucosa has been shown to be a risk factor for gastric cancer (Schorah et al., American Journal of Clinical Nutrition 53 (Supplement1): 287S-2935S, 1991 and Reed et al., Iarc Scientific Publications, 105:139-142, 1991). In patients suffering from dyspepsia, chronic gastritis, hypochlorhydria, and duodenal cancer, the intragastric concentrations of vitamin C were significantly lower (Sobala et al., Gastroenterology 97(2):357-363, 1989 and O'Conner et al., Gut 30(4):436-442, 1989

### SUMMARY OF THE INVENTION

According to the present invention, there is provided a chewing gum, toothpaste, dental paint, viscous gel, aerosol, gargling gel or mouth rinse comprising a bismuth compound for use as a medicament in a topical dosage form for the treatment of halitosis in an oral cavity, comprising a pharmaceutically acceptable bismuth compound in a topical dosage form which provides controlled release of the bismuth compound in the oral cavity for at least 5 minutes in a therapeutically-effective amount of at least 2 times the minimum inhibitory concentration for Helicobacter Pylori, wherein the bismuth compound is selected from the group consisting of colloidal bismuth subcitrate, bismuth subcitrate, bismuth citrate, bismuth salicylate, bismuth subsalicylate, bismuth subcarbonate, bismuth tartrate, bismuth subgallate, tripotassium dicitrato bismuthate, bismuth aluminate, bismuth polysulfates, bismuth polyhydroxy compounds, alpha-D-glucopyranoside bismuth complex, beta-D-fructofuranosyl-oktakis (hydrogen sulfate) bismuth complex, L-dihydro ascorbyl-tetrakis (hydrogen sulfate) bismuth complex, dihydro diascorbyl urea amide-deca(hydrogen sulfate) bismuth complex, bismuth ascorbyl sulfate, bismuth subascorbate, and cyclodextrin bismuth sulfate.

According to a further aspect of the present invention there is provided a bismuth compound in the manufacture of a medicament in topical dosage form for treating halitosis, wherein the bismuth compound is selected from the group consisting of colloidal bismuth subcitrate, bismuth subcitrate, bismuth citrate, bismuth salicylate, bismuth subsalicylate, bismuth subcarbonate, bismuth tartrate, bismuth subgallate, tripotassium dicitrato bismuthate, bismuth aluminate, bismuth polysulfates, bismuth polyhydroxy compounds, alpha-D-glucopyranoside bismuth complex, beta-D-fructofuranosyl-oktakis (hydrogen sulfate) bismuth complex, L-dihydro ascorbyl-tetrakis (hydrogen sulfate) bismuth complex, dihydro diascorbyl urea amide-deca(hydrogen sulfate) bismuth complex, bismuth ascorbyl sulfate, bismuth subascorbate, and cyclodextrin bismuth sulphate, wherein topical dosage form provides controlled release of the bismuth compound in the oral cavity for at least 5 minutes in a therapeutically-effective amount of at least 2 times the minimum inhibitory concentration for Helicobacter Pylori.

The invention provides oral topical dosage forms with pharmaceutically usable bismuth compounds and/or antibacterial compounds and/or antibiotics that eradicate or reduce H. Pylori in dental plaque. The invention further provides a treatment with bismuth compounds and/or antibacterial compounds and/or antibiotics which are effective against Campylobacter rectus and Treponema denticola which are responsible for causing halitosis.

### BRIEF DESCRIPTION OF THE DRAWINGS

Figure 1 is a generalized reaction diagram for the synthesis of bismuth sulfates.
Figure 2 is a graph of human saliva concentration versus time which shows the release of bismuth from CBS chewing gum.

### DETAILED DESCRIPTION OF THE INVENTION

Therefore, bismuth compound used in this invention should be a pharmaceutically acceptable antimicrobacterial agent against H. Pylori, such as colloidal bismuth subcitrate (CBS), bismuth subcitrate, bismuth citrate, bismuth salicylate, bismuth subsalicylate, bismuth subnitrate, bismuth subcarbonate, bismuth tartrate, bismuth subgallate, tripotassium dicitrato bismuthate and bismuth aluminate. Preferably, colloidal bismuth subcitrate (CBS), tripotassium dicitrato bismuthate, bismuth subcitrate, bismuth subsalicylate and their combination are chosen. More preferably, CBS and tripotassium dicitrato bismuthate are chosen. And further selection is made for CBS.

The structural formula of CBS is:

[Bi(OH)₃]₃BiC₆H₆O₇(1,2,3-PROPANETRICARBONIC ACID, 2-HYDROXY, BISMUTH(3T)POTASSIUM; CAS#57644-54-9

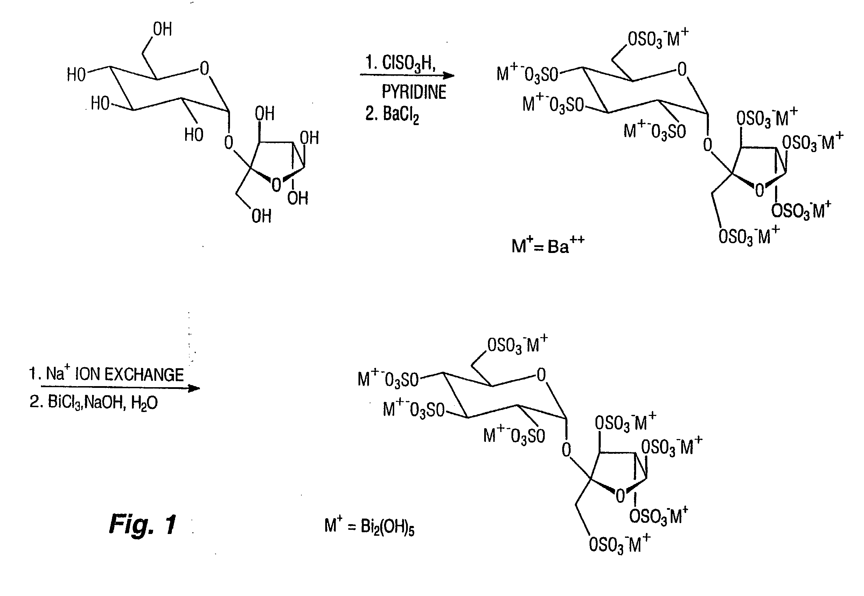

Other bismuth-containing compounds which are useful in the present invention are those described in Bos et al., U.S. Patent No. 4,801,608 and in Serfortein, U.S. Patent No. 4,153,685. Other bismuth compounds, namely complexes of polysulfates, of polyhydroxy compounds such as sugars, sugar alcohols, and ascorbic acid and its derivatives, as well as alpha-D-glucopyranoside bismuth complex, beta-D-fructofuranosyl-oktakis (hydrogen sulfate) bismuth complex, are used in and I-dihydro ascorbyl-tetrakis (hydrogen sulfate) bismuth complex are used in the present invention. A generalized reaction diagram for the synthesis of bismuth sulfates is shown in Figure 1. These compounds will deliver bismuth more effectively and will have less side effects in treating H. Pylori. The compounds will lend themselves to controlled release oral dosage forms and oral topical dosage forms for eradication of H. Pylori in dental plaque.

Chemical structures of the compounds used in this invention are illustrated below:

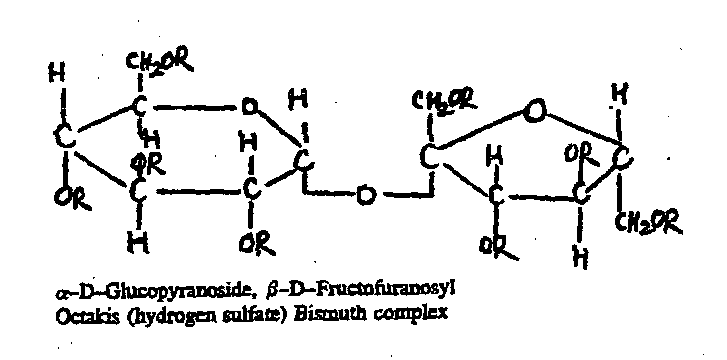

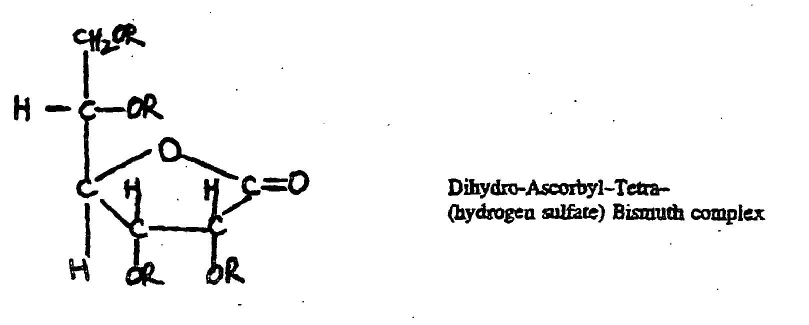

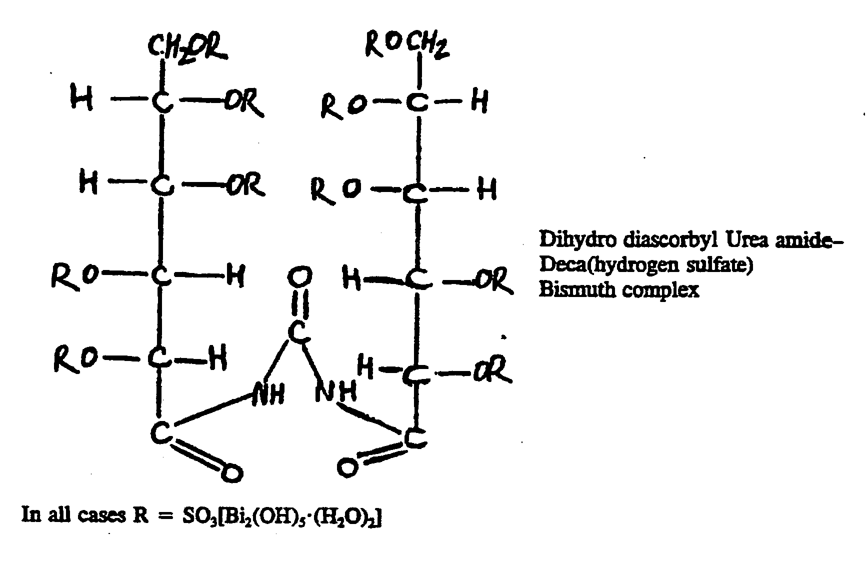

The ascorbic acid-derived molecules are synthesized in a manner completely analogous to the reaction diagram for synthesis of bismuth sulfates set forth above.

In addition to antibacterial bismuth-compounds, antibiotics can be used for reduction/elimination of Helicobacter pylori in the oral cavity. Antibiotics useful herein include, but are not limited to, Tetracycline, Amoxicillin, Ampicillin, Doxycycline, Erythromycin, Clarithromycin, Metronidazole, Tinidazole, Ciproflaxacin, Oflaxacin, Norflaxacin, Furazolidine, Nitrofurantoin. Antibacterials useful herein include, but are not limited to naturally occurring peptides and synthetic peptide antibacterials such as Lanthocins, and particularly, Nicin and related peptides, Proton pump inhibitors such as Omeprazole and Lansoprazole, Sanguinaria and other antibacterials obtained from plant sources, as well as bismuth-containing compounds.

The present methods utilize topical oral dosage forms to deliver bismuth compounds, antibiotics, and/or antibacterials directly to the oral cavity in concentrations sufficient to reduce or eliminate H. Pylori in an oral cavity.

Each oral-topical dosage form of this invention is the one which can make it possible to release bismuth compound and/or antibacterial compounds and/or antibiotics into oral cavity in a predictable manner and result in appropriate antibacterial concentration. Those examples of such forms include a chewing-gum form, dental paints, viscous gels, a toothpaste form, a gargling-gel form and mouth-rinse form. Preferably, a chewing-gum form is chosen. Further, a chewing-gum form is most preferred due to its easy-to-use characteristic, predictable drug release and increased drug contact with dental surface. The chewing gum delivery system especially enables sustained contact of the antibacterial agents with the entire oral cavity and therefore, enhance bactericidal/bacteriostatic efficacy. We have already demonstrated that chewing gum formulation containing an antibacterial agent, colloidal bismuth subcitrate, releases the drug in a precise and reproducible fashion during a 15 minute chewing time. Viscous gel formulations, and dental paint formulations also will be able to provide sustained concentration of antibacterial agents in the oral cavity.

Oral-topical dosage forms containing bismuth in this invention must release enough bismuth, antibiotic, and/or antibacterial into saliva for eradication of H. Pylori in the oral cavity. The minimum inhibitory concentration (MIC) of bismuth for H. Pylori varies in each bismuth compound. For instance, it is reported that the MIC of CBS for H. Pylori is 8 mg/L and its range is 4 to 32 mg/L.

Therefore, the dosage form should release bismuth into saliva up to at least two times the MIC. preferably a minimum of 2 to 10 times, most preferably 2 to 250 times. In order to achieve this level of release rate, the bismuth content per dosage form should be about 50 mg to 200 mg, preferably a minimum of 10 mg to 50 mg, most preferably 25 mg to 50 mg. For instance, each piece of CBS-containing chewing gum should contain approximately 50 mg to 200 mg of CBS, preferably a minimum of 10 mg to 50 mg, most preferably 25 mg to 50 mg.

Time release of each dosage form in this invention must be long enough to eradicate H. Pylori. Although the duration of time release varies in each bismuth compound and in each dosage form, it is desirable that at least 25 % of the dose is released within 2 minutes, more preferably at least 35%, more preferably at least 45%, more preferably at least 55%, more preferably at least 65%, most preferably at least 75%, more preferably within 2 to 15 minutes, most preferably within 10 to 15 minutes.

In other preferred embodiments of the invention presented herein, a chewing gum drug delivery system is utilized to provide sustained concentration of bismuth compounds, antibiotics, and/or antibacterial compounds which are proven antibacterial agents against H. Pylori and anti-plaque agents to help these compounds penetrate the dental plaques to reach the site of H. Pylori infection. The chewing gum delivery system enables sustained contact of the antibacterial agents with the entire oral cavity and therefore, enhances bacteriocidal efficacy.

Where antibiotic and/or antibacterial agents other than bismuth are to be used, oral-topical dosage form in this invention must release enough antibiotic/antibacterial into saliva for eradication of H. Pylori from the oral cavity. The minimum inhibitory concentration (MIC) varies for each antibiotic/ antibacterial agent. However, for most of the antibiotics listed in this invention, the MIC values are between less than 1 to 10 mcg/mL, or 1 to 10 mg/L.

Therefore, the topical-oral dosage from should release the antibacterial agent into saliva up to at least 2 times the MIC, preferably a minimum of 2 to 10 times, most preferably 2 to 100 times. In order to achieve this level of release, the antibacterial content per unit of dosage form should be about 10 to 100 mg, preferably a minimum of 5 to 50 mg, most preferably 10 to 25 mg. For instance, each piece of chewing gum should contain approximately 10 to 100 mg. of the antibiotic or antibacterial agent. Preferably a minimum of 5 to 50 mg, most preferably 10 to 25 mg.

The topical-oral dosage forms of this invention must release the antibiotic/antibacterial over an extended time. The duration of release should be at least 5 minutes, preferably 10 minutes, most preferably 15 minutes. Further, at least 25 % of the dose is released within 5 minutes, more preferably at least 35 %, more preferably at least 45%, more preferably at least 55%, more preferably at least 65 %, most preferably at least 75% of the antibiotics/antibacterial content should be released within 5 minutes, preferably within 2 to 15 minutes, most preferably within 10 to 15 minutes.

In other preferred embodiment of this invention, a chewing gum delivery system is utilized to provide sustained concentration of antibiotic/antibacterial agent several times above its MIC for H. Pylori over at least 10 times.

The anti-plaque agents further contribute to improved efficacy by breaking down the plaque and exposing the bacterial colonies to the antibacterial agents. The chewing gum formulation containing CBS, antibiotic, and/or antibacterial releases the drug in a precise and reproducible fashion during a 15-minute chewing time. Anti-plaque agents include, but are not limited to, glucanase anhydroglucosidase, glucose oxidase, silicon oil, sanguinarine, and the like. Chewing gum formulations may optionally include crystalline sorbitol, sorbitol solution, mannitol, Nova-base^{™}, or any other gum base, dextrans, cellulose derivatives, buffer salts, sweeteners, flavors, and the like.

Optionally, metronidazole can be added to CBS chewing gum to broaden the antimicrobial activity against H. Pylori.

### Example 1 ― Preparation of_Therapeutic Substance

To an aqueous solution of ammonia are added bismuth citrate, citric acid, and caustic potash in specific stoichiometric proportions, and at specific temperatures. The solution is examined for turbidity and, if required, additional volume of ammonia solution is added to render the solution clear. The solution is then filtered on a carbon bed and spray dried to obtain free-flowing powder material. The product is packaged in an air and moisture proof glass container.

### Example 2 ― Preparation of Topical Dosage Form

Brief general description of the preferred topical dosage form, chewing gum, is set forth as follows. Fully melt the gum base (at approximately 90°C) in Brabender mixer, a jacketed mixer with sigma blades. Remove the hot water from the mixer jacket, allow to cool, and add lecithin and mix well. Cool further to approximately 50°C, and add liquid flavor and mannitol. Mix until uniform. Dry blend colloidal bismuth subcitrate in sorbitol, and blend sodium citrate in sorbo syrup. Add sorbitol and sorbo syrup blends to the gum base. Cool the product to 35 °C, add flavor and sweetener and mix until smooth. Remove the product from the mixing kettle, roll to form a sheet of uniform thickness and score to produce chewing gum sticks weighing 2.5 g each. Wrap individual gum sticks in aluminum foil and place in plastic bags. Where the gum is to include antibiotic or antibacterial compounds, the agent is coated with a polymeric substance to mask any untoward taste or odor, and to further regulate its release in the saliva.

### Example 3 ― Composition of CBS-Containing Gum

Two variations of the 50 mg CBS gum (Table 1) were used. Both formulations used were identical with the exception that Formula-2 contained sodium citrate to impart a firmer texture, while Formula-1 did not.

**Table 1 FORMULATIONS OF THE GUM (APPROX. 2.5 gm A PIECE)**

| Formula-1 | | Formula-2 | |
|---|---|---|---|
| CBS | 50.0mg | CBS | 50.0mg |
| Crystalline Sorbitol | 910.0 | Crystalline Sorbitol | 910.0 |
| Gum Base | 575.0 | Gum Base | 575.0 |
| Sorbitol Solution | 500.0 | Sorbitol Solution | 500.0 |
| Mannitol | 400.0 | Mannitol | 400.0 |
| Peppermint Oil | 25.0 | Peppermint Oil | 25.0 |
| Spray Dried Peppermint | 12.5 | Spray Dried Peppermint | 2.5 |
| Grade t Lecithin | 10.0 | Grade t Lecithin | 10.0 |
| Aspartame | 10.0 | Aspartame | 10.0 |
| Sodium Citrate | 10.0 | | |
| | 2502.5mg | Total: | 2492.5mg |

Colloidal Bismuth Subcitrate (CBS) and other bismuth compounds, including bismuth subcitrate, bismuth citrate, bismuth salicylate, bismuth subsalicylate, bismuth subnitrate, bismuth subcarbonate, bismuth tartrate, bismuth subgallate, tripotassium dicitrato bismuthate and bismuth aluminate. Preferably, colloidal bismuth subcitrate (CBS), tripotassium dicitrato bismuthate, bismuth subcitrate, bismuth subsalicylate are coated with the following coating agent to regulate their dissolution and salivary release: bee's wax, carnauba wax, shellac, cellulose acetate phthalate, methyl cellulose, propyl cellulose, hydroxy propylcellulose, ethyl cellulose, hydroxy propylmethylcellulose, ethylcellulose, polymethyl methacrylate, and Eudragit® polymers, polyvinyl pyrohidone, polyvinyl alcohol, etc.

Moderately water soluble bismuth compounds such as bismuth ascorbyl sulfate, bismuth sucrose sulfate, bismuth subascorbate, cyclodextrin bismuth sulfate are used in the chewing gum dosage form to produce sustained concentration in the saliva.

Synthetic and natural latex-based chewing gum bases are used to tightly enclose bismuth compounds and other antibacterial/antibiotic compounds to cause their gradual release in the saliva.

These formulation/composition modifications are designed to:
(1) provide control release of antibacterial/antibiotic compounds to increase their bactericidal efficacy against oral cavity/dental plaque bound H. Pylori; and
(2) avoid/minimize oral cavity discoloration/blackening caused by quick or instant release of bismuth compounds in the saliva.

### Example 4 ― Measurement of Release Rate of Bismuth into Saliva

Among six healthy human subjects, who gave informed consent, three chewed the CBS-containing gum with sodium citrate, and the other three chewed CBS-containing gum without sodium citrate. The subjects chewed the gum samples for a total of 15 minutes. Saliva samples were collected at time interval of 0, 1, 5, 10, and 15 minutes of chewing. The saliva samples were then submitted to an analytical laboratory for bismuth analysis. Results are shown in Table 2.

**Table 2 IN VIVO SALIVARY CONCENTRATION OF CBS FROM THE CHEWING GUM**

| Formula | chewing time (min.) | saliva vol. (mL) | conc of Bi (ppm) | conc of active CBS (u/mL) | X MIC |
|---|---|---|---|---|---|
| formula-1 | 0 | 4.4(±0.5) | | | |
| | 1 | 3.3(±1.4) | 900.7(±239.1) | 1270.3(±334.7) | 148.7(±42.0) |
| | 5 | 5.4(±1.5) | 257.7(±112.3) | 363.3(±158.9) | 45.0(±19.9) |
| | 10 | 4.9(±1.3) | 28.0 (±5.0) | 40.0 (±6.6) | 5.0 (±1.0) |
| | 15 | 5.2(±2.1) | 15.8 (±17.8) | 25.7 (±23.0) | 3.1 (±2.7) |
| | | | | | |
| formula-2 | 0 | 7.2(±0.5) | | | |
| | 1 | 4.8(±1.9) | 888.3(±329.5) | 1257.0(±464.5) | 156.3(±58.0) |
| | 5 | 8.5(±1.7) | 326.0(±113.3) | 572.7(±159.7) | 63.7(±19.9) |
| | 10 | 7.5(±3.4) | 30.0 (±9.5) | 42.3 (±13.6) | 5.0 (±1.7) |
| | 15 | 7.7(±3.8) | 10.7 (±6.7) | 14.7 (±9.2) | 1.8 (±1.2) |

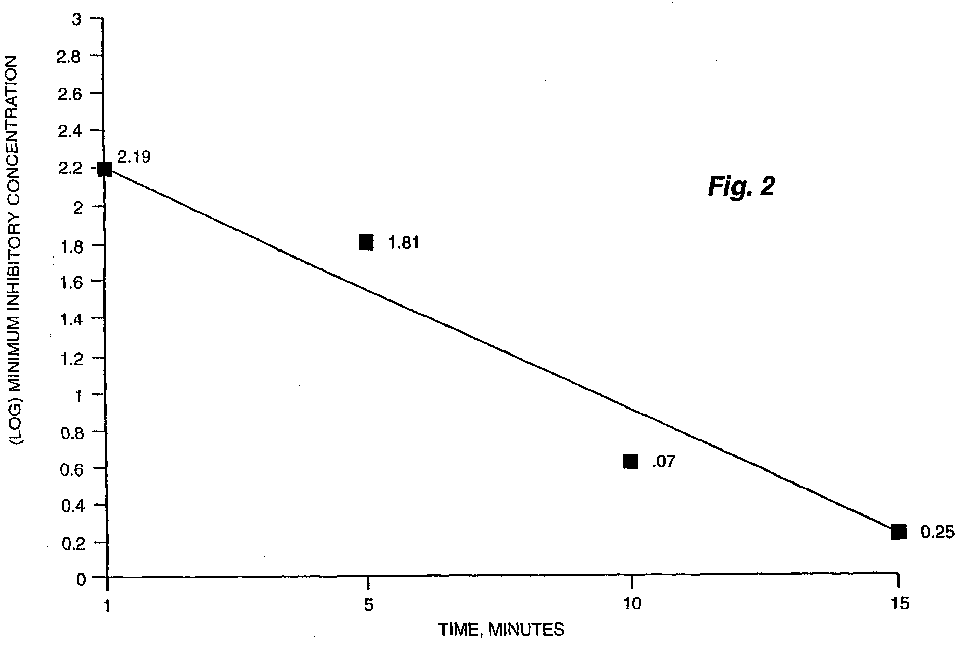

Saliva samples were analyzed for elemental bismuth in ppm units. The results were then converted to mg of active CBS per mL of saliva and also expressed as a multiple of minimum inhibitory concentration (MIC) of CBS for H. Pylori. As can be seen from the results (formula-2 of Table-3), the salivary concentrations of CBS are 156, 64, 5, and 1.8 times the MIC at 1, 5, 10 and 15 minutes, respectively. The constant bathing of the oral cavity from saliva containing sufficient concentration of CBS (2 to 5 times the MIC) for up to 15 minutes can be expected to further reduce the viable cells of H. Pylori. These results are plotted in Figure 2 which shows a graph of human saliva concentration versus time.

### Example 5 ― Sensory Analysis of Chewing Gum

Sensory characteristics of the chewing gum were evaluated by the subjects during the 15 minutes of chewing. Again, three subjects chewed the CBS gum containing sodium citrate and three subjects chewed the CBS gum without sodium citrate. A nine point rating scale was used to evaluate each category (Tables 3 and 4).

**Table 3 RESULTS OF SENSORY ANALYSIS RATING OF CBS GUM WITHOUT SODIUM CITRATE (Formula-1)**

| | CHEWING TIME | | | |
|---|---|---|---|---|
| SENSORY CHARACTERISTICS | 1MIN | 5MIN | 10 MIN | 15 MIN |
| Overall Flavor | 6.3 | 6.0 | 5.3 | 5.0 |
| (0= dislike extremely, 8 = like extremely) | (±1.2) | (±1.0) | (±1.5) | (±1.0) |
| | | | | |
| Flavor Intensity | 5.7 | 4.7 | 3.7 | 3.0 |
| (0 = none, 8 = very strong) | (±1.5) | (±1.2) | (±0.6) | (±1.0) |
| | | | | |
| Chew Qualities | 6.0 | 6.0 | 5.3 | 5.0 |
| (0 = dislike extremely, 8 = like extremely) | (±1.0) | (±1.0) | (±1.5) | (±1.0) |
| | | | | |
| Unpleasant Aftertaste | 0.0 | 0.0 | 0.0 | 0.0 |
| (0 = none, 8 = very strong) | (±0.0) | (±0.0) | (±0.0) | (±0.0) |
| | | | | |
| Overall Qualities | 6.3 | 6.0 | 5.7 | 5.3 |
| (0 = dislike extremely, 8 = like extremely) | (±1.2) | (±1.0) | (±1.2) | (±1.5) |

**Table 4 RESULTS OF SENSORY ANALYSIS RATING OF CBS GUM WITH SODIUM CITRATE (Formula-2)**

| | CHEWING TIME | | | |
|---|---|---|---|---|
| SENSORY CHARACTERISTICS | 1MIN | 5MIN | 10 MIN | 15 MIN |
| Overall Flavor | 6.7 | 5.7 | 4.7 | 4.7 |
| (0 = dislike extremely, 8 = like extremely) | (±0.6) | (± 1.5) | (±1.2) | (±1.2) |
| | | | | |
| Flavor Intensity | 6.7 | 6.0 | 5.0 | 3.7 |
| (0 = none, 8 = very strong) | (±0.6) | (±0.0) | (±1.0) | (±1.5) |
| | | | | |
| Chew Qualities | 4.7 | 5.0 | 4.3 | 4.3 |
| (0= dislike extremely, 8 = like extremely) | (±2.1) | (±2.0) | (±1.5) | (±0.6) |
| | | | | |
| Unpleasant Aftertaste | 0.7 | 1.7 | 1.7 | 2.0 |
| (0 = none, 8 = very strong) | (±1.2) | (±2.1) | (±2.1) | (±2.0) |
| | | | | |
| Overall Qualities | 6.3 | 5.7 | 4.7 | 4.0 |
| (0 = dislike extremely, 8 = like extremely) | (±0.6) | (±1.2) | (±1.2) | (±1.0) |

In general, there were no dramatic differences in the sensory analysis between the two formulas. The sensory panel clearly shows that both chewing gum formulations have a desirable level of flavor and taste, and cause a minimal unpleasant aftertaste after chewing.

### Example 6 ― Topical Safety

Topical safety was evaluated in the six volunteers for up to 60 minutes after administration of the gum. The subjects were asked to report any adverse effects such as discomfort or irritation in the oral cavity.

There were no reports of any discomfort or irritation in the oral cavity by any of the subjects at either the 15 or 60 minute post administration time periods.

### Example 7 ― Storage Stability Study

Samples of CBS-containing gum (50mg) were wrapped individually in foil wrappers. The sticks of gum were then placed in foil laminate bags, sealed, and placed in storage. Storage conditions include 40 °C and room temperature (RT). The duration of the stability testing was 90 days. The results are shown in Tables 5-8 below.

**Table 5. THREE MONTH STABILITY DATA IN VIVO SALIVARY CONCENTRATIONS IN HUMAN SUBJECTS OF CBS FROM THE 50 MG CBS CHEWING GUM (Mfg. August 1993, Batch No. CBS-50CG-0002)**

| **TIME/CONDITION** | **CHEWING TIME (min)** | **SALIVA VOLUME (mL)** | **CONC OF Bi (ppm)** | **CONC OF B1 (µg/mL)** | **CONC OF ACTIVE CBS (µg/mL)** | **X MIC** |
|---|---|---|---|---|---|---|
| **ZERO TIME** | 0 | 4.2 (±1.6) | NA | NA | NA | NA |
| | 1 | 4.9 (±4.5) | 1937.3 (±753.5) | 1937.3 (±753.5) | 2729.0 (±1060.2) | 341.0 (±132.7) |
| | 5 | 6.4 (±3.1) | 437.0 (±152.1) | 437.0 (±152.1) | 615.7 (±214.5) | 77.0 (±26.9) |
| | 10 | 3.9 (±0.1) | 36.0 (±28.6) | 36.0 (±28.6) | 50.7 (±40.5) | 6.4 (±5.0) |
| | 15 | 4.5 (±1.3) | 5.0 (±4.6) | 5.0 (±4.6) | 7.0 (±6.6) | 0.9 (±0.8) |
| **3 MONTHS/ 40°C** | 0 | 5.6 (±1.4) | NA | NA | NA | NA |
| | 1 | 2.9 (±1.8) | 1922.3 (±511.8) | 1922.3 (±511.8) | 2710.0 (±791.9) | 338.6 (±90.3) |
| | 5 | 5.6 (±1.7) | 399.3 (±278.1) | 363.7 (±113.3) | 563.0 (±329.3) | 70.3 (±49.1) |
| | 10 | 5.3 (±1.4) | 25.7 (±11.4) | 30.0 (±9.5) | 362.0 (±160.5) | 45.4 (±20.1) |
| | 15 | 4.9 (±0.4) | 7.9 (±4.9) | 10.7 (±6.7) | 10.8 (±6.8) | 1.4 (±0.9) |
| **3 MONTHS/ ROOM TEMP.** | 0 | 5.1 (±1.3) | NA | NA | NA | NA |
| | 1 | 4.1 (±1.5) | 1240.0 (±458.7) | 1240.0 (±458.7) | 1748.0 (±646.6) | 218.0 (±80.6) |
| | 5 | 7.2 (±2.3) | 518.7 (±118.7) | 518.7 (±118.7) | 731.3 (±167.6) | 91.0 (±21.8) |
| | 10 | 6.0 (±2.2) | 12.5 (±10.6) | 12.5 (±10.6) | 17.7 (±14.6) | 2.1 (±1.8) |
| | 15 | 5.6 (±1.6) | 4.5 (±2.2) | 4.5 (±2.2) | 6.0 (±2.6) | 0.7 (±0.3) |

| | | | | | | |
|---|---|---|---|---|---|---|
| n = 3 for each group | | | | | | |

**Table 6. THREE MONTH STABILITY DATA RESULTS OF SENSORY ANALYSIS RATING OF 50 MG CBS GUM (Mfg. August 1993, Batch No. CBS-50CG-0002)**

| | | **CHEWING TIME** | | | |
|---|---|---|---|---|---|
| | **SENSORY CHARACTERISTIC** | **1 MIN** | **5 MIN** | **10 MlN** | **15 MIN** |
| **ZERO TIME** | OVERALL FLAVOR | 6.7 (±0.6) | 6.3 (±0.6) | 5.3 (±0.6) | 5.3 (±0.6) |
| | FLAVOR INTENSITY | 6.3 (±1.2) | 5.3 (±1.2) | 4.0 (±1.0) | 4.0 (±1.0) |
| | CHEW QUALITIES | 6.7 (±0.6) | 6.3 (±0.6) | 5.7 (±0.6) | 5.3 (±0.6) |
| | UNPLEASANT AFTERTASTE | 0.0 (±0.0) | 0.0 (±0.0) | 0.0 (±2.1) | 0.0 (±0.0) |
| | OVERALL QUALITIES | 6.7 (±0.6) | 6.3 (±0.6) | 5.7 (±0.6) | 5.3 (±1.2) |
| **3 MONTHS/ 40°C** | OVERALL FLAVOR | 6.0 (±0.0) | 4.7 (±0.6) | 2.7 (±1.2) | 2.7 (±1.2) |
| | FLAVOR INTENSITY | 5.3 (±1.2) | 3.0 (±0.0) | 2.3 (±0.6) | 2.0 (±1.0) |
| | CHEW QUALITIES | 5.7 (±0.6) | 5.0 (±1.0) | 4.3 (±0.6) | 4.3 (±0.6) |
| | UNPLEASANT AFTERTASTE | 0.3 (±0.6) | 0.3 (±0.6) | 0.0 (±0.0) | 0.0 (±0.0) |
| | OVERALL QUALITIES | 6.0 (±0.0) | 4.3 (±0.6) | 2.7 (±0.6) | 2.3 (±0.6) |
| **3 MONTHS/ ROOM TEMP.** | OVERALL FLAVOR | 6.3 (±0.6) | 6.3 (±0.6) | 5.3 (±0.6) | 4.3 (±0.6) |
| | FLAVOR INTENSITY | 5.7 (±1.5) | 5.3 (±1.5) | 4.3 (±1.5) | 4.0 (±1.7) |
| | CHEW QUALITIES | 6.0 (±1.0) | 6.0 (±1.0) | 5.3 (±0.6) | 4.3 (±0.6) |
| | UNPLEASANT AFTERTASTE | 0.0 (±0.0) | 0.0 (±0.0) | 0.0 (±0.0) | 0.0 (±0.0) |
| | OVERALL QUALITIES | 6.3 (±0.6) | 6.3 (±0.6) | 5.3 (±0.6) | 4.7 (±1.2) |

| | | | | | |
|---|---|---|---|---|---|
| Note: n = 3 for each analysis | | | | | |
| Rating Scale: 0=dislike extremely, 9-like extremely for: Overall Flavor, Chew Quality, Overall Quality 0=none, 9-like extremely for: Flavor Intensity 0=none, 9=very strong for: Unpleasant Aftertaste | | | | | |

**TABLE 7. EXPONENTIAL REGRESSION DATA OF TIME VS SALIVARY CONCENTRATIONS EXPRESSED AS X MIC**

| | **Initial Test Lot # CBS-50-CG-0001** | **Stability Lot # CBS-50CG-0002** | | | **Clinical Lot # CBS-50CG-0003** |
|---|---|---|---|---|---|
| | | **Zero Time** | **3 mo./RT** | **3 mo./40°C** | |
| A (intercept) | 240.0 | 563.1 | 575.7 | 422.3 | 446.5 |
| b (slope) | -0.339 | -0.432 | -0.361 | -0.448 | -0.426 |
| r (correlation coefficient) | 0.992 | 0.998 | 0.948 | 0.971 | 0.959 |
| K (pseudo first order rate constant) | -0.339 | 0.432 | 0.361 | 0.448 | 0.426 |
| t_{0.5} (min.) | 2.04 | 1.60 | 1.92 | 1.55 | 1.63 |

| | | | | | |
|---|---|---|---|---|---|
| Mean t_{0.5} = 1.748 (±0.218) | | | | | |

**TABLE 8. RELEASE OF CBS FROM THE CHEWING GUM AFTER 15 MINUTES OF CHEWING BY HUMAN SUBJECTS**

| | **Stability Lot # CBS -50CG-0002** | | | **Clinical Lot** # **CBS-50CG-0003** |
|---|---|---|---|---|
| | **Zero Time** | **3 mo./RT** | **3 mo./40°C** | |
| Mg CBS/2.5 g gum Before chewing (%) | 45.6 (100) | 44.5 (100) | 46.1 (100) | 46.2 (100) |
| Mg CBS/2.5 g gum After 15 min chewing (%) | 3.5 (7.6) | 4.0 (9.0) | 4.5 (9.8) | 3.8 (100) |

| | | | | |
|---|---|---|---|---|
| Mean % of CBS Remaining in the gum after 15 min of chewing = 8.6 (±1.0) | | | | |

Each stick of the gums used for the stability study (1 for zero-time, 2 for three month, total 3 sticks) was from the same lot number. The results show that bismuth concentration remains stable over the tested time period.

### Example 8 ― Denture Material Exposure Study

An evaluation of CBS salivary concentration on various denture materials was conducted in order to test any potential staining effect of the CBS on denture materials. Artificial saliva was used (Table 9).

**Table 9 THE COMPOSITION OF ARTIFICIAL SALIVA**

| Ingredients | Concentration per Liter |
|---|---|
| Sodium Bicarbonate | 0.50 g |
| Sodium Phosphate, Dibasic, Dihydrate | 0.85 g |
| Calcium Chloride | 0.44 g |
| Magnesium Chloride | 0.06 g |
| Potassium Chloride | 1.40 g |
| Sodium Carboxyl Methyl Cellulose | 2.00 g |
| Phosphoric Acid to adjust pH to 6.4 Distilled Water | QS |

The test saliva was prepared by dissolving 0.500 g of colloidal bismuth subcitrate in 100 mL of the above artificial saliva. 500 mL of Artificial Saliva (RT) was placed in one of two identical glass jars with lids. In the other jar was placed 500 mL of the Artificial Saliva (RT) containing 0.50% of CBS. In each of the jars the denture material block and a magnetic stirrer was placed. The jars were then placed on the magnetic platform and set to agitate at a minimum rate. The denture materials that were exposed to artificial saliva containing CBS or placebo included (Table 10).

**Table 10 DENTURE MATERIALS**

| | |
|---|---|
| 1) | Natural tooth with silver amalgam filling |
| 2) | Composite resin (used on anterior teeth for filling) |
| 3) | Denture base acrylic resin |
| 4) | Porcelain fused to metal |
| 5) | Partial denture metal frame |
| 6) | Acrylic tooth (artificial) |
| 7) | Natural tooth |

The four hour exposure of natural tooth and other denture materials to 0.5% CBS in artificial saliva with mild agitation did not cause any staining, discoloration, or changes in texture.

### Example 9 ― Clinical Efficacy Data

An open label, placebo-controlled pilot clinical study in ten patients with initial positive response for H. Pylori in the dental plaque has been initiated. Data from six patients (four patients treated with CBS 50 mg chewing gum six times-a-day and two patients treated with placebo chewing gum six times-a-day for fifteen days) has been obtained. The dental plaque samples from the patients were collected before treatment, day 7 and day 15 after treatment, and tested by microbiological culture and CLO test. The results are set forth in Table 11 below:

**Table 11**

| | | | | | |
|---|---|---|---|---|---|
| TREATED GROUP (n = 4) | | | | | |

| | | CLO | DUR POSITIVE (HRS:MINS) | CULTURE | SIDE EFFECTS (Stain/Odor) |
|---|---|---|---|---|---|
| Pt 1 | Day 0 | + | 1:00 | + | NE |
| 30/M | Day 7 | + | 1:45 | -ve | - |
| | Day 15 | + | 1:30 | -ve | - |
| Pt 2 | Day 0 | + | 2:15 | + | NE |
| 42/M | Day 7 | + | 1:30 | NA | - |
| | Day 15 | + | 4:00 | -ve | - |
| Pt 3 | Day 0 | + | 2:30 | + | NIL |
| 31/M | Day 7 | + | 4:30 | NA | NIL |
| | Day 15 | + | 5:30 | NA | NIL |
| Pt 4 | Day 0 | + | 2:30 | NA | NIL |
| 29/F | Day 7 | + | 4:00 | NA | NIL |
| | Day 15 | + | 5:30 | NA | NIL |
| Mean CLO response time after 15 days = 4.125 HR | | | | | |
| PLACEBO (n = 2) | | | | | |

| | | CLO | DUR POSITIVE (HRS:MINS) | CULTURE | SIDE EFFECTS (Stain/Odor) |
|---|---|---|---|---|---|
| Pt 1 | Day 0 | + | 1:00 | NA | NIL |
| 26/M | Day 7 | + | 1:30 | NA | NIL |
| | Day 15 | + | 1:30 | NA | NIL |
| Pt 2 | Day 0 | + | 1:15 | NA | NIL |
| 28/M | Day 7 | + | 2:00 | NA | NIL |
| | Day 15 | + | 2:30 | NA | NIL |
| Mean CLO response time after 15 days = 2.0 HR | | | | | |

| | | | | | |
|---|---|---|---|---|---|
| NA = Not available | | | | | |
| NE = Not evaluated (before chewing) | | | | | |

The data show that for patients treated with CBS 50 mg chewing gum and placebo chewing gum on day 15 the mean CLO response times are 4.125 hours and 2.0 hours, respectively. The longer CLO test response time for CBS 50 mg chewing gum group compared to the placebo chewing gum group is indicative of substantial reduction in H. Pylori density in the oral cavity of the active treatment group.

### Example 10 ― Toxicology

A number of animal toxicity studies and human clinical investigations have demonstrated safety of bismuth compounds, especially CBS, in therapeutic dose ranges. No toxicity has been reported in chronic daily administration of high doses of CBS (160, 320, and 640 mg/kg body weight representing 2, 4, and 8 times the human therapeutic dose respectively) in rats treated for three months or dogs treated for six months. See Wieriks et al., Journal of Gastroenterology 17 (Supplement 80):11-16 (1982).

Long term safety of CBS and treatment of peptic ulcers at a standard dose of 480 mg (expressed as bismuthtrioxide) in four daily divided doses has been examined by Bader, Digestion 37(Supplement 2):53-59 (1987). CBS was first introduced in Europe in 1971 and since that time 1.5 million treatments have been dispensed. During eight years of use of CBS tablets [De-Nol^{(R)}] in Europe between 1978 and 1986 under a more comprehensive adverse reaction monitoring system, only 13 adverse reaction forms were completed. Five of these adverse reactions were ascribed to CBS: one case of headache, one case of stomach pain, one case of diarrhea, and two cases of allergy (mainly in the form of skin rashes). A high degree of safety of CBS in therapeutic applications for the treatment of peptic ulcers is reported in a recent review of pharmacology of bismuth-containing compounds by Lambert, Review of Infectious Diseases 13(Supplement 8):691-695 (1991). In reviewing safety and pharmacokinetics of CBS, Bennet, Scandinavian Journal of Gastroenterology 26(Supplement 185):29-35 (1991), has calculated the systemic bioavailability of bismuth after oral dosing of CBS to be in the range of 0.16 to 0.28 % of the administered dose, and concluded that steady-state blood levels of 50-100 mg/mL are unlikely to cause any neurotoxicity.

### Example 11 ― Composition of Antibiotic or Antibacterial-Containing Gum

The chewing gum formulation comprises antibiotic or antibacterial agents in concentration ranges from 10 to 50 mg per piece of gum. The chewing gum-base consists of Crystalline Sorbitol, Gum Base, Sorbitol Solution, Mannitol, Peppermint Oil, Spray Dried Peppermint, Grade t Lecithin, Aspartame, and Sodium Citrate, as set forth in Table 1 above. The formulation may also contain Glucanase, Anhydroglucosidase, Glucose oxidase, Silicon oil, Sanguinarine and related compounds as anti-plaque agents. Caboxy methyl cellulose, Hydroxy propyl methyl cellulose, Polyethylene glycol, Poly methyl methacrylates, Acrylic acid copolymers and other polymers as coating agents.

Saliva samples are analyzed for antibiotic or antibacterial agents in ppm units. The results are then converted to mg of active agent per mL of saliva and also expressed as a multiple of minimum inhibitory concentration (MIC) of the agent for H. Pylori. The salivary concentrations of the agent are 156, 64, 5, and 1.8 times the MIC at 1, 5, 10 and 15 minutes, respectively. The constant bathing of the oral cavity from saliva containing sufficient concentration of the agent (2 to 5 times the MIC) for up to 15 minutes can be expected to further reduce the viable cells of H. Pylori. These results are plotted to show a graph of human saliva concentration versus time.

Sensory characteristics of the chewing gum are evaluated by the subjects during the 15 minutes of chewing. Again, three subjects chews the gum containing sodium citrate and three subjects chewed the gum without sodium citrate. A nine point rating scale is used to evaluate each category.

In general, there are no dramatic differences in the sensory analysis between the two formulas. The sensory panel shows that both chewing gum formulations have a desirable level of flavor and taste, and cause a minimal unpleasant aftertaste after chewing.

Topical safety is evaluated in the six volunteers for up to 60 minutes after administration of the gum. The subjects are asked to report any adverse effects such as discomfort or irritation in the oral cavity.

There are no reports of any discomfort or irritation in the oral cavity by any of the subjects at either the 15 or 60 minute post administration time periods.

Samples of the agent-containing gum (50mg) are wrapped individually in foil wrappers. The sticks of gum are then placed in foil laminate bags, sealed, and placed in storage. Storage conditions include 40°C and room temperature (RT). The duration of the stability testing is 90 days.

Each stick of the gums used for the stability study (1 for zero-time, 2 for three month, total 3 sticks) is from the same lot number. The results show that bismuth concentration remains stable over the tested time period.

An evaluation of salivary concentration of the agent on various denture materials is conducted in order to test any potential staining effect of the CBS on denture materials. Artificial saliva is used (Table 9).

The test saliva is prepared by dissolving 0.500 g of the antibiotic or antibacterial agent in 100 mL of the above artificial saliva. 500 mL of Artificial Saliva (RT) is placed in one of two identical glass jars with lids. In the other jar is placed 500 mL of the Artificial Saliva (RT) containing 0.50% of the agent. In each of the jars the denture material block and a magnetic stirrer is placed. The jars are then placed on the magnetic platform and set to agitate at a minimum rate. The denture materials that are were exposed to artificial saliva containing the agent or placebo are included. The four hour exposure of natural tooth and other denture materials to 0.5% of the agent in artificial saliva with mild agitation does not cause any staining, discoloration, or changes in texture.

To assess clinical efficacy, patients with positive response for the presence of H. Pylori in the dental plaque/oral cavity are divided into two treatment groups. Group I is given placebo chewing gum to be chewed 2 or 6 times a day for 2 or 4 weeks. Group II is given chewing gum containing antibiotic/antibacterial agent to be chewed 2 or 6 times a day for 2 or 4 weeks. Patient's dental plaque/saliva samples are collected at time 0 (Pre-treatment) on days 7, 14, 28, and tested for H. Pylori presence and density. The incidence of H. Pylori presence in the 29 placebo group and the active treatment group is compared. The group receiving the chewing gum containing antibiotic/antibacterial shows significantly lower incidence of H. Pylori presence in the dental plaque/saliva compared to placebo chewing gum group after 2 and 4 weeks of treatment.

### Example 12 ― Antibacterial Efficacy for Treatment of Halitosis

Campylobacter rectus, Helicobacter pylori, and Treponema denticola have been demonstrated to be associated with Halitosis (bad breath). The compounds and methods of the present invention, including CBS as well as ascorbyl bismuth derivative, have demonstrated in vitro activity against all three bacteria, as indicated by their minimum effective concentrations (MICs) presented in Table 12 below.

**Table 12**

| Test Organisms | Bismuth Ascorbyl Sulfate (µg/mL) | Bismuth Sucrose Sulfate (µg/mL) | CBS (µg/mL) (µg/mL) |
|---|---|---|---|
| Campylobacter rectus | 256 | > 256 | 256 |
| Helicobacter pylori | 8 | 16 | 2 |
| Treponema denticala | 16 | 32 | 32 |

## Claims

1. A chewing gum, toothpaste, dental paint, viscous gel, aerosol, gargling gel or mouth rinse comprising a bismuth compound for use as a medicament in a topical dosage form for the treatment of halitosis in an oral cavity, comprising a pharmaceutically acceptable bismuth compound in a topical dosage form which provides controlled release of the bismuth compound in the oral cavity for at least 5 minutes in a therapeutically-effective amount of at least 2 times the minimum inhibitory concentration for Helicobacter Pylori, wherein the bismuth compound is selected from the group consisting of colloidal bismuth subcitrate, bismuth subcitrate, bismuth citrate, bismuth salicylate, bismuth subsalicylate, bismuth subcarbonate, bismuth tartrate, bismuth subgallate, tripotassium dicitrato bismuthate, bismuth aluminate, bismuth polysulfates, bismuth polyhydroxy compounds, alpha-D-glucopyranoside bismuth complex, beta-D-fructofuranosyl-oktakis (hydrogen sulfate) bismuth complex, L-dihydro ascorbyl-tetrakis (hydrogen sulfate) bismuth complex, dihydro diascorbyl urea amide-deca(hydrogen sulfate) bismuth complex, bismuth ascorbyl sulfate, bismuth subascorbate, and cyclodextrin bismuth sulfate.

2. The chewing gum, toothpaste, dental paint, viscous gel, aerosol, gargling gel or mouth rinse of Claim 1, wherein the topical oral dosage form provides controlled release of the bismuth compound in the oral cavity in an amount of at least 10 times the minimum inhibitory concentration for Helicobacter pylori.

3. The chewing gum, toothpaste, dental paint, viscous gel, aerosol, gargling gel or mouth rinse of Claims 1 or 2, which is a chewing gum.

4. The chewing gum, toothpaste, dental paint, viscous gel, aerosol, gargling gel or mouth rinse of Claim 1, wherein the bismuth compound is colloidal bismuth subcitrate and wherein the dosage form is a chewing gum.

5. The chewing gum, toothpaste, dental paint, viscous gel, aerosol, gargling gel or mouth rinse of any one of Claims 1 to 4 wherein the topical oral dosage form further comprises a pharmaceutically effective amount of metronidazole.

6. The chewing gum, toothpaste, dental paint, viscous gel, aerosol, gargling gel or mouth rinse of any one of Claims 1 to 5, wherein the topical oral dosage form further comprises an anti-plaque agent.

7. The chewing gum, toothpaste, dental paint, viscous gel, aerosol, gargling gel or mouth rinse of Claim 6, wherein the anti-plaque agent is glucanase anhydroglycosidase, glucose oxidase, silicon oil, or sanguinarine.

8. The chewing gum, toothpaste, dental paint, viscous gel, aerosol, gargling gel or mouth rinse of claim 1, wherein the bismuth compound is selected from the group consisting of colloidal bismuth subcitrate, bismuth subcitrate, bismuth citrate, bismuth salicylate, bismuth subsalicylate, bismuth subcarbonate, bismuth tartrate, bismuth subgallate, tripotassium dicitrato bismuthate, bismuth aluminate, and dihydro diascorbyl urea amide-deca(hydrogen sulfate) bismuth complex.

9. The use of a bismuth compound in the manufacture of a medicament in topical dosage form for treating halitosis, wherein the bismuth compound is selected from the group consisting of colloidal bismuth subcitrate, bismuth subcitrate, bismuth citrate, bismuth salicylate, bismuth subsalicylate, bismuth subcarbonate, bismuth tartrate, bismuth subgallate, tripotassium dicitrato bismuthate, bismuth aluminate, bismuth polysulfates, bismuth polyhydroxy compounds, alpha-D-glucopyranoside bismuth complex, beta-D-fructofuranosyl-oktakis (hydrogen sulfate) bismuth complex, L-dihydro ascorbyl-tetrakis (hydrogen sulfate) bismuth complex, dihydro diascorbyl urea amide-deca(hydrogen sulfate) bismuth complex, bismuth ascorbyl sulfate, bismuth subascorbate, and cyclodextrin bismuth sulphate, wherein topical dosage form provides controlled release of the bismuth compound in the oral cavity for at least 5 minutes in a therapeutically-effective amount of at least 2 times the minimum inhibitory concentration for Helicobacter Pylori.

10. Use as claimed in Claim 9, wherein the bismuth compound is selected from the group consisting of bismuth polysulfates, bismuth polyhydroxy compounds, alpha-D-glucopyranoside bismuth complex, beta-D-fructofuranosyl oktakis (hydrogen sulfate) bismuth complex, L-dihydro ascorbyltetrakis (hydrogen sulfate) bismuth complex, bismuth ascorbyl sulfate, bismuth subascorbate, and bismuth cyclodextrin sulfate.

## Patentansprüche

1. Kaugummi, Zahnpasta, Zahnlack, viskoses Gel, Aerosol, Gurgelgel oder Mundspülung, umfassend eine Bismutverbindung, zur Verwendung als ein Arzneimittel in einer topischen Dosierungsform für die Behandlung von Mundgeruch in einer Mundhöhle, umfassend eine pharmazeutisch akzeptable Bismutverbindung in einer topischen Dosierungsform, welche für mindestens 5 Minuten eine kontrollierte Freisetzung der Bismutverbindung in der Mundhöhle in einer therapeutisch wirksamen Menge von mindestens dem Zweifachen der minimalen inhibitorischen Konzentration für Helicobacter Pylori ermöglicht,
worin die Bismutverbindung ausgewählt ist aus der Gruppe bestehend aus kolloidalem Bismutsubcitrat, Bismutsubcitrat, Bismutcitrat, Bismutsalicylat, Bismutsubsalicylat, Bismutsubcarbonat, Bismuttartrat, Bismutsubgallat, Trikaliumdicitratobismutat, Bismutaluminat, Bismutpolysulfaten, Bismutpolyhydroxyverbindungen, alpha-D-Glucopyranosid-Bismutkomplex, beta-D-Fructofuranosyl-oktakis (Hydrogensulfat)-Bismutkomplex, L-Dihydroascorbyl-tetrakis (Hydrogensulfat)-Bismutkomplex, Dihydrodiascorbyl-Hamstoffamiddeca (Hydrogensulfat)-Bismutkomplex, Bismutascorbylsulfat, Bismutsubascorbat und Cyclodextrinbismutsulfat.

2. Kaugummi, Zahnpasta, Zahnlack, viskoses Gel, Aerosol, Gurgelgel oder Mundspülung nach Anspruch 1, worin die topische Dosierungsform eine kontrollierte Freisetzung der Bismutverbindung in der Mundhöhle in einer Menge von mindestens dem Zehnfachen der minimalen inhibitorischen Konzentration für Helicobacter Pylori ermöglicht.

3. Kaugummi, Zahnpasta, Zahnlack, viskoses Gel, Aerosol, Gurgelgel oder Mundspülung nach Anspruch 1 oder 2, welches ein Kaugummi ist.

4. Kaugummi, Zahnpasta, Zahnlack, viskoses Gel, Aerosol, Gurgelgel oder Mundspülung nach Anspruch 1, worin die Bismutverbindung kolloidales Bismutsubcitrat ist und worin die Dosierungsform ein Kaugummi ist.

5. Kaugummi, Zahnpasta, Zahnlack, viskoses Gel, Aerosol, Gurgelgel oder Mundspülung nach einem der Ansprüche 1 bis 4, worin die topische orale Dosierungsform weiterhin eine pharmazeutisch wirksame Menge Metronidazol umfasst.

6. Kaugummi, Zahnpasta, Zahnlack, viskoses Gel, Aerosol, Gurgelgel oder Mundspülung nach einem der Ansprüche 1 bis 5, worin die topische orale Dosierungsform weiterhin ein Mittel gegen Plaque umfasst.

7. Kaugummi, Zahnpasta, Zahnlack, viskoses Gel, Aerosol, Gurgelgel oder Mundspülung nach Anspruch 6, worin das Mittel gegen Plaque Glucanaseanhydroglycosidase, Glucoseoxidase, Siliconöl oder Sanguinarin ist.

8. Kaugummi, Zahnpasta, Zahnlack, viskoses Gel, Aerosol, Gurgelgel oder Mundspülung nach Anspruch 1, worin die Bismutverbindung ausgewählt ist aus der Gruppe bestehend aus kolloidalem Bismutsubcitrat, Bismutsubcitrat, Bismutcitrat, Bismutsalicylat, Bismutsubsalicylat, Bismutsubcarbonat, Bismuttartrat, Bismutsubgallat, Trikaliumdicitratobismutat, Bismutaluminat und einem Dihydrodiascorbyl-Harnstoffamid-deca (Hydrogensulfat)-Bismutkomplex.

9. Verwendung einer Bismutverbindung bei der Herstellung eines Arzneimittels in einer topischen Dosierungsform für die Behandlung von Mundgeruch, worin die Bismutverbindung ausgewählt ist aus der Gruppe bestehend aus kolloidalem Bismutsubcitrat, Bismutsubcitrat, Bismutcitrat, Bismutsalicylat, Bismutsubsalicylat, Bismutsubcarbonat, Bismuttartrat, Bismutsubgallat, Trikaliumdicitratobismutat, Bismutaluminat, Bismutpolysulfaten, Bismutpolyhydroxyverbindungen, alpha-D-Glucopyranosid-Bismutkomplex, beta-D-Fructofuranosyl-oktakis (Hydrogensulfat)-Bismutkomplex, L-Dihydroascorbyl-tetrakis (Hydrogensulfat)-Bismutkomplex, Dihydrodiascorbyl-Harnstoffamid-deca (Hydrogensulfat)-Bismutkomplex, Bismutascorbylsulfat, Bismutsubascorbat und Cyclodextrinbismutsulfat, worin die topische Dosierungform eine kontrollierte Freisetzung der Bismutverbindung in der Mundhöhle für mindestens 5 Minuten in einer therapeutisch wirksamen Menge von mindestens dem Zweifachen der minimalen inhibitorischen Konzentration für Helicobacter Pylori ermöglicht.

10. Verwendung nach Anspruch 9, worin die Bismutverbindung ausgewählt ist aus Bismutpolysulfaten, Bismutpolyhydroxyverbindungen, alpha-D-Glucopyranosid-Bismutkomplex, beta-D-Fructofuranosyl-oktakis (Hydrogensulfat)-Bismutkomplex, L-Dihydroascorbyl-tetrakis (Hydrogensulfat)-Bismutkomplex, Bismutascorbylsulfat, Bismutsubascorbat und Bismutcyclodextrinsulfat.

## Revendications

1. Gomme à mâcher, pâte dentifrice, vernis dentaire, gel visqueux, aérosol, gel pour gargarisme ou bain de bouche comprenant un composé de bismuth, destiné à être utilisé comme médicament dans une forme posologique topique pour le traitement de l'haleine fétide dans la cavité buccale, comprenant un composé de bismuth pharmaceutiquement acceptable dans une forme posologique topique qui provoque la libération contrôlée du composé de bismuth dans la cavité buccale pendant au moins 5 minutes en une quantité thérapeutiquement efficace égale à au moins 2 fois la concentration inhibitrice minimale pour Helicobacter pylori, le composé de bismuth étant choisi dans le groupe consistant en le sous-citrate de bismuth colloïdal, le sous-citrate de bismuth, le citrate de bismuth, le salicylate de bismuth, le sous-salicylate de bismuth, le sous-carbonate de bismuth, le tartrate de bismuth, le sous-gallate de bismuth, le dicitratobismuthate tripotassique, l'aluminate de bismuth, des polysulfates de bismuth, des composés polyhydroxyliques de bismuth, un complexe alpha-D-glucopyrannoside-bismuth, un complexe bêta-D-fructofurannosyl-octakis(hydrogénosulfate)-bismuth, un complexe L-dihydro-ascorbyl-tétrakis(hydrogénosulfate)-bismuth, un complexe dihydro-diascorbyl-urée-amide-déca(hydrogénosulfate)-bismuth, l'ascorbylsulfate de bismuth, le sous-ascorbate de bismuth et le sulfate de bismuth-cyclodextrine.

2. Gomme à mâcher, pâte dentifrice, vernis dentaire, gel visqueux, aérosol, gel pour gargarisme ou bain de bouche suivant la revendication 1, dans lequel la forme posologique orale topique provoque la libération contrôlée du composé de bismuth dans la cavité buccale en une quantité égale à au moins 10 fois la concentration inhibitrice minimale pour Helicobacter pylori.

3. Gomme à mâcher, pâte dentifrice, vernis dentaire, gel visqueux, aérosol, gel pour gargarisme ou bain de bouche suivant la revendication 1 ou 2, qui est une gomme à mâcher.

4. Gomme à mâcher, pâte dentifrice, vernis dentaire, gel visqueux, aérosol, gel pour gargarisme ou bain de bouche suivant la revendication 1, dans lequel le composé de bismuth est le sous-citrate de bismuth colloïdal, et dans lequel la forme posologique est une gomme à mâcher.

5. Gomme à mâcher, pâte dentifrice, vernis dentaire, gel visqueux, aérosol, gel pour gargarisme ou bain de bouche suivant l'une quelconque des revendications 1 à 4, dans lequel la forme posologique orale topique comprend en outre une quantité pharmaceutiquement efficace de métronidazole.

6. Gomme à mâcher, pâte dentifrice, vernis dentaire, gel visqueux, aérosol, gel pour gargarisme ou bain de bouche suivant l'une quelconque des revendications 1 à 5, dans lequel la forme posologique orale topique comprend en outre un agent antiplaque.

7. Gomme à mâcher, pâte dentifrice, vernis dentaire, gel visqueux, aérosol, gel pour gargarisme ou bain de bouche suivant la revendication 6, dans lequel l'agent antiplaque est la glucanase anhydroglycosidase, la glucose-oxydase, une huile de silicone ou la sanguinarine.

8. Gomme à mâcher, pâte dentifrice, vernis dentaire, gel visqueux, aérosol, gel pour gargarisme ou bain de bouche suivant la revendication 1, dans lequel le composé de bismuth est choisi dans le groupe consistant en sous-citrate de bismuth colloïdal, sous-citrate de bismuth, citrate de bismuth, salicylate de bismuth, sous-salicylate de bismuth, sous-carbonate de bismuth, tartrate de bismuth, sous-gallate de bismuth, dicitrato-bismuthate tripotassique, aluminate de bismuth et un complexe dihydro-diascorbyl-urée-amide-déca(hydrogénosulfate)-bismuth.

9. Utilisation d'un composé de bismuth dans la production d'un médicament sous une forme posologique topique pour le traitement de l'haleine fétide, dans laquelle le composé de bismuth est choisi dans le groupe consistant en sous-citrate de bismuth colloïdal, sous-citrate de bismuth, citrate de bismuth, salicylate de bismuth, sous-salicylate de bismuth, sous-carbonate de bismuth, tartrate de bismuth, sous-gallate de bismuth, dicitrato-bismuthate tripotassique, aluminate de bismuth, polysulfates de bismuth, composés polyhydroxyliques de bismuth, complexe alpha-D-glucopyrannoside-bismuth, complexe bêta-D-fructofurannosyl-octakis(hydrogénosulfate)-bismuth, complexe L-dihydro-ascorbyl-tétrakis(hydrogénosulfate)-bismuth, complexe dihydro-diascorbyl-urée-amide-déca(hydrogénosulfate)-bismuth, ascorbylsulfate de bismuth, sous-ascorbate de bismuth et sulfate de bismuth-cyclodextrine, la forme posologique topique provoquant la libération contrôlée du composé de bismuth dans la cavité buccale pendant au moins 5 minutes en une quantité thérapeutiquement efficace égale à au moins 2 fois la concentration inhibitrice minimale pour Helicobacter pylori.

10. Utilisation suivant la revendication 9, dans laquelle le composé de bismuth est choisi dans le groupe consistant en polysulfates de bismuth, composés polyhydroxyliques de bismuth, complexe alpha-D-glucopyrannoside-bismuth, complexe bêta-D-fructofurannosyl-octakis(hydrogénosulfate)-bismuth, complexe L-dihydro-ascorbyl-tétrakis(hydrogénosulfate)-bismuth, ascorbylsulfate de bismuth, sous-ascorbate de bismuth et sulfate de bismuth-cyclodextrine.
